(19) Europäisches Patentamt
European Patent Office
Office européen des brevets

(11) Publication number : **0 491 562 A1**

(12) **EUROPEAN PATENT APPLICATION**

(21) Application number : **91311743.8**

(22) Date of filing : **18.12.91**

(51) Int. Cl.[5] : **C07D 491/10,** A61K 31/445, // (C07D491/10, 307:00, 221:00)

(30) Priority : **19.12.90 US 629797**

(43) Date of publication of application : **24.06.92 Bulletin 92/26**

(84) Designated Contracting States : **AT BE CH DE DK ES FR GB GR IT LI LU NL SE**

(71) Applicant : **FISONS CORPORATION**
**755 Jefferson Road**
**Rochester, NY 14623 (US)**

(72) Inventor : **Griffith, Ronald Conrad**
**41 Northfield Gate**
**Pittsford, New York 14534 (US)**
Inventor : **Wu, Edwin Shen-Chou**
**147 Ashley Drive**
**Rochester, New York 14620 (US)**

(74) Representative : **Wright, Robert Gordon McRae**
**FISONS plc 12 Derby Road**
**Loughborough Leicestershire LE11 0BB (GB)**

# (54) Novel spirofurane derivatives.

(57) Compounds of formula I,

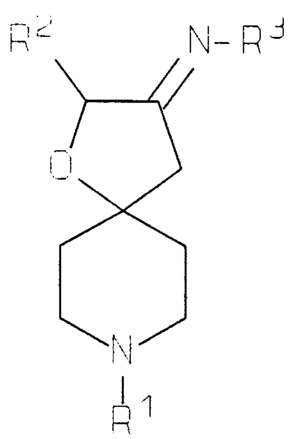

I

wherein

$R^1$ represents hydrogen or alkyl $C_{1-3}$,

$R^2$ represents hydrogen or alkyl $C_{1-6}$, alkenyl $C_{3-6}$ or alkynyl $C_{3-6}$,

$R^3$ represents $OCH_3$, $OCONR^4R^5$ or $NHCOOR^6$,

$R^4$, $R^5$, and $R^6$ represent hydrogen or $C_{1-6}$ alkyl, provided that when $R^3$ represents $OCH_3$, then $R^1$ and $R^2$ both represent methyl,

and salts and solvates thereof, are useful as pharmaceuticals, in particular they possess cholinergic properties and are useful in the treatment of neurological disorders.

EP 0 491 562 A1

This invention relates to novel spirofurane derivatives, processes for their preparation, pharmaceutical compositions containing them, and to their usefulness in the treatment of neurological and mental illnesses.

According to the cholinergic hypothesis of memory impairment in Alzheimer's disease, senile dementia and age-associated memory impairment, a deficiency of cholinergic function plays a major role in the progressive development of the disease. This has led to the belief that enhancement of muscarinic cholinergic transmissions at cerebral cortical sites would be beneficial for treatment of the disease.

International Patent Application No. WO 90/15804 discloses a large group of spirofurane derivatives which are indicated in the treatment of various neurological disorders. We have now found a small group of spirofurane derivatives which are particularly useful in the treatment of neurological and mental illnesses.

According to the invention there are provided compounds of formula I,

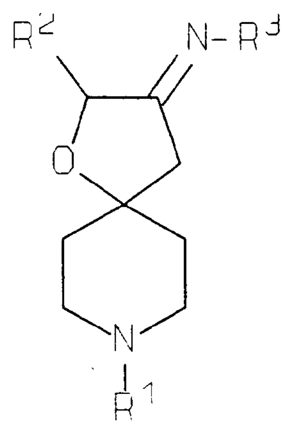

I

wherein,

$R^1$ represents hydrogen or alkyl $C_{1-3}$,

$R^2$ represents hydrogen or alkyl $C_{1-6}$, alkenyl $C_{3-6}$ or alkynyl $C_{3-6}$,

$R^3$ represents $OCH_3$, $OCONR^4R^5$ or $NHCOOR^6$,

$R^4$, $R^5$, and $R^6$ represent hydrogen or $C_{1-6}$ alkyl, provided that when $R^3$ represents $OCH_3$, then $R^1$ and $R^2$ both represent methyl,

and salts and solvates thereof.

It will be appreciated that salts of the compounds for use in medicine will be non-toxic pharmaceutically acceptable salts. Other salts may, however, be useful, in the preparation of the compounds of formula I or their non-toxic pharmaceutically acceptable salts. Acid addition salts, for example, may be formed by mixing a solution of the compound of formula I with a solution of a pharmaceutically acceptable non-toxic acid such as hydrochloric acid, fumaric acid, maleic acid, succinic acid, carbonic acid or phosphoric acid.

Solvates of the compounds of formula I and their salts include in particular, hydrates of the salts, for example, hemihydrates and monohydrates.

Alkyl groups which $R^1$ may represent include methyl, ethyl, n-propyl and iso-propyl.

Alkyl groups which $R^2$, $R^4$, $R^5$ and $R^6$ may represent include methyl, ethyl, n-propyl, iso-propyl, n-butyl, isobutyl, s-butyl and t-butyl.

Alkenyl groups which $R^2$ may represent include 2-propenyl, 2-butenyl and 2-methyl-2-propenyl.

Alkynyl groups which $R^2$ may represent include 2-propynyl and 2-butynyl.

We prefer compounds of formula I or pharmaceutically acceptable salts thereof, in which:

$R^1$ represents hydrogen or methyl, preferably methyl; $R^2$ represents hydrogen or methyl, preferably methyl; $R^3$ represents OCH3; $R^4$ represents hydrogen or methyl, preferably methyl; $R^5$ represents hydrogen or methyl, preferably methyl; and $R^6$ represents methyl or ethyl, preferably ethyl.

Substituents which are not readily inactivated by hydrolysis in vivo are also preferred.

Many of the compounds of the present invention have at least one asymmetric centre and can therefore exist as enantiomers, and in some cases as diastereomers. Some of the compounds of the present invention contain carbon-carbon double bonds, and all contain a carbon-nitrogen double bond: they can therefore exist as isomers. It is to be understood that the invention covers all such isomers and mixtures thereof.

Compounds in which the carbon-nitrogen double bond has the syn (equivalent to Z) configuration are preferred.

The compounds of the present invention are useful because they possess pharmacological activity in animals. In particular, the compounds stimulate central muscarinic acetylcholine receptors as can be demonstrated in studies of the affinity constants of the compounds for [$^3$H]-oxotremorine-M binding sites in rat cortical membrane preparations. The compounds may therefore be useful in the treatment of neurological and mental illnesses whose clinical manifestations are due to involvement of specific populations of cholinergic neurones. Such diseases include memory impairment, presenile and senile dementia (also known as Alzheimer's disease and senile dementia of the Alzheimer type respectively), Huntington's chorea, tardive dyskinesia, hyperkinesia, mania and Tourette Syndrome. The compounds are also useful analgesic agents and therefore useful in the treatment of severe painful conditions such as rheumatism, arthritis, and terminal illness.

Biochemical procedures for measuring affinity and estimating efficacy at brain muscarinic receptors are indicative of the potential utilities for these compounds.

Based largely on the selectivity of the antagonist, pirenzepine, muscarinic receptors have been classified as M1 (high affinity for pirenzepine) and M2 (low affinity). Brain receptor subtypes appear pharmacologically similar to those in peripheral ganglia (M1) and heart (M2). Receptor subtypes are coupled preferentially to different second messengers and ion channels. Thus in brain as well as other tissues, M1 receptors stimulate phosphatidyl inositol(PI) hydrolysis while M2 receptors inhibit adenylate cyclase. From the results of animal experiments, it is suggested that muscarinic agonists having M1 receptor selectivity may be advantageous in improving impaired performance, memory retention and other clinical manifestations of senile dementia.

Two binding assays are used to estimate the affinity and predict the efficacy of test compounds at muscarinic receptors in rat cerebral cortex.

The procedure is described in detail by Freedman, SB, Harley, EA, and Iversen LL, in Br. J. Pharmacol. 93: 437-445 (1988). A rat brain crude membrane preparation is incubated with a radiolabeled antagonist [([$^3$H]N-Methyl- scopolamine) (NMS)] or agonist [([$^3$H]Oxotremorine-M) (Oxo-M)] and various concentrations of test compound at 30°C for 40 and 60 minutes, respectively. The membranes are then collected by vacuum filtration on filters and receptor bound radioactivity is determined by liquid scintillation spectroscopy. The affinities (Ki) of the test compound for the agonist high affinity state (radiolabeled agonist) and high and low agonist affinity states together (radiolabeled antagonist) are determined from the competition binding curves using a nonlinear iterative curve fitting computer program. The ratio of measured dissociation constants (the Ki's) are also used as an index of agonist efficacy. Full agonists such as carbachol exhibit an antagonist/agonist ratio of >1800. Partial agonists show a range of ratios extending from 20 to 1500. Antagonists have ratios from 1 to 10.

Compounds of the formula I with a high affinity for the Oxo-M binding site with a Ki of less than 1$^{\mu m}$ and preferably less than 0.1μm and a NMS-Ki/Oxo-M-Ki ratio of greater than 100 are preferred.

In a procedure for measuring agonist efficacy at M1 muscarinic receptors in rat brain hippocampus, the muscarinic cholinergic agonist activity of a test compound is measured using an in vitro M1 muscarinic receptor linked enzyme assay which measures the formation of inositol phosphate from phosphatidyl inositol. The assay procedure is described in detail by Fisher SK and Bartus RT, J. Neurochem. 45: 1085-1095(1985). Rat brain hippocampal tissue was cross sliced into 350*350 μm segments which were equilibrated for one hour at 37°C in oxygenated Krebs-Hensleit buffer. Aliquots of slices were then incubated with [$^3$H]myo-inositol, lithium chloride, and various concentrations of test compound for 120 minutes in a 95% $O_2$/5% $CO_2$ atmosphere at 37°C. The reaction is terminated by addition of chloroform/methanol solution (1:2, v/v) and the [$^3$H]inositol phosphates were extracted into the aqueous phase. [$^3$H]Inositol phosphates were purified by ion exchange chromatography using Dowex AG-1x8 anion exchange resin in the formate form. Inositol phosphates were selectively eluted from the resin with a 1M ammonium formate 0.1M formic acid solution. Tritium was determined by liquid scintillation spectroscopy. The magnitude of stimulation of inositol phosphate formation by high concentrations of full agonists such a carbachol was assigned a value of 100%. Partial agonists produced a maximal rate of inositol phosphate formation which varied, according to the compound, from 10 to 80%. Weak partial agonists and antagonists did not stimulate the formation of inositol phosphates.

Compounds of formula I with a maximal rate of inositol phosphate formation of greater than 15% are preferrred.

Partial agonists identified in the above assays may be tested for any selectivity for M1 versus M2 receptors. A measure of M2-receptor mediated inhibition of adenylate cyclase in rat heart membranes can be obtained according to procedures described by Ehlert, F.J. et al [J Pharmacol. Exp. Ther. 228:23-29(1987)].

Some of the compounds may possess muscarinic antagonist properties and thus may be useful as antisecretory agents in the management of peptic ulcers and acute rhinitis, or in the treatment of motion sickness and parkinsonism.

A measure of the effects of cholinergic drugs on memory and performance can be obtained in vivo by evaluating the effects of drugs on memory and motor performance of rats according to the procedures described by Ordy, J.M., et al.; An Animal Model of Human-Type Memory Loss Based on Aging, Lesion, Forebrain

Ischemia, and Drug Studies With the Rat; Neurobiology of Aging, Volume 9, pp. 669-683 (1988). Compounds with muscarinic agonist activity such as arecoline, for example, enhanced memory in old rats, without effects on performance.

Thus according to another aspect of the invention, there is provided the use of a compound of formula I, as defined above, or a pharmaceutically acceptable salt thereof, as a pharmaceutical.

According to yet another aspect of this invention, there is provided the use of a compound of formula I, as defined above, or a pharmaceutically acceptable salt thereof, in the manufacture of a medicament for use in the treatment of neurological disorders.

The compounds of the invention may be administered by any convenient route, eg orally, parenterally or rectally. The daily dose required will of course vary with the particular compound used, the particular condition being treated and with the severity of that condition. However, in general a total daily dose of from about 0.1 to 10 mg/kg of body weight, preferably about 0.1 to 1 mg/kg is suitable, administered from 1 to 4 times a day.

The compound of formula I will generally be administered in the form of a suitable pharmaceutical composition. Thus according to another aspect of the invention, there is provided a pharmaceutical composition comprising a compound of formula I, or a pharmaceutically acceptable salt thereof, in admixture with a pharmaceutically acceptable carrier.

The pharmaceutical composition is preferably in unit dose form. Such forms include solid dosage forms, eg tablets, pills, capsules, powders, granules, and suppositories for oral, parenteral or rectal administration, and liquid dosage forms, eg sterile parenteral solutions or suspensions, suitably flavoured syrups, flavoured emulsions with edible oils such as cottonseed oil, sesame oil, coconut oil and peanut oil, and elixirs and similar pharmaceutical vehicles.

Solid compositions may be prepared by mixing the active ingredient with pharmaceutical carriers, eg conventional tabletting ingredients such as corn starch, lactose, sucrose, sorbitol, talc, stearic acid, magnesium stearate, dicalcium phosphate, gums and other diluents, eg water, to form a homogeneous preformulation composition in which the active ingredient is uniformly dispersed so that it may be readily subdivided into equally effective unit dosage forms containing typically from 0.1 to about 500mg of the active ingredient. The solid dosage forms may be coated or otherwise compounded to prolong the action of the composition.

In order to reduce unwanted peripherally mediated side effects, it may be advantageous to include in the composition a peripherally acting cholinergic antagonist (or anti-muscarinic agent) such as N-methylscopolamine, N-methylatropine, propantheline, methantheline or glycopyrrolate.

The compounds of the invention have the advantage that they are more potent, have a longer duration of action, have a broader range of activity, have fewer side effects, are more stable, or have other more useful properties than prior art compounds previously used to treat neurological and mental illnesses.

According to the invention, there is also provided a process for the preparation of compounds of formula I or pharmaceutically acceptable salts or solvates thereof, which comprises

a) preparing a compound of formula I in which $R^3$ represents $OCONR^4R^5$ and $R^4$ and $R^5$ are as defined above,

by reacting the corresponding compound of formula I in which $R^3$ is OH with the corresponding aminocarbonyl halide, or

b) preparing a compound of formula I in which $R^3$ represents $OCH_3$ or $NHCOOR^6$ and $R^6$ is as defined above,

by reacting the corresponding compound of formula II

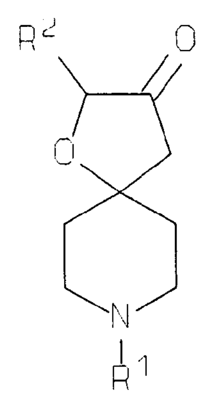

II

in which $R^1$ and $R^2$ are as defined above with the corresponding nitrogen nucleophile of the formula $H_2N$-

$OCH_3$ or $H_2NNHCOOR^6$, or

c) removing a protecting group from a compound of formula I in which the amino group is protected, or

d) preparing a pharmaceutically acceptable salt of a compound of formula I by treating a compound of formula I with an appropriate acid or preparing a free base of a compound of formula I by treating the acid addition salt with a stronger base.

In the reaction of process (a), conventional acylation techniques for amines may be used, for example, the reactions may be carried out in the absence of a solvent; however, a suitable inert solvent may be used, for example, toluene, methylene chloride or tetrahydrofuran. The reactions may be carried out in the presence of a base, for example, sodium hydride or pyridine. The reactions may be carried out at a temperature of, for example, from 0°-100°C.

The reaction of process (b) may be carried out with the nitrogen nucleophile in a suitable solvent at a temperature of, for example, from 0°-100°C. Protic solvents are preferred, for example, methanol or ethanol. The reaction may be carried out in the presence of an acid, for example, hydrogen chloride.

In the reaction of process (c), removal of the protecting group depends on the nature of the protecting group and includes acidic or basic cleavage or hydrogenolysis. Suitable amine protecting groups are, for example, ethoxycarbonyl, benzyloxycarbonyl, t-butyloxycarbonyl or $C_{1-3}$ alkanoyl. Further protecting groups and methods for their removal are described in T. W. Greene, Protecting Groups in Organic Synthesis, Wiley Interscience, 1981.

In process (d), the salts may be formed by reacting the free base, or a salt or derivative thereof with one or more equivalents of the appropriate acid. The reaction may be carried out in a solvent in which the salt is insoluble or in which the salt is soluble or in mixtures of the solvents.

The starting materials for the reaction of process (a) can be prepared from the corresponding ketones of formula II by reaction with hydroxylamine essentially according to the procedures described for method (b) above.

The starting ketones of formula II can be prepared by a variety of methods, for example

e) oxidation of the corresponding alcohol compound.

Conventional oxidation techniques for secondary alcohols may be used in a suitable solvent. A preferred oxidizing agent is that of a mixture of oxalyl chloride and dimethylsulfoxide in a suitable inert solvent, for example, methylene chloride. The reaction may be carried out at a temperature of, for example, from -80°-30°C.

f) reacting the corresponding compound in which $R^1$ is a protecting group and the carbonyl group is protected as a ketal with a reducing agent and subsequent deprotection by hydrolysis of the ketal.

Ketal protection and deprotection of the ketone is effected by conventional methods, for example, with 1,2-dihydroxy ethane in the presence of an acid, for example, p-toluene sulfonic acid to form the ketal and by aqueous acid hydrolysis for removal of the ketal group. Reduction of the nitrogen protecting group, for example, a $C_{1-3}$ alkanoyl or alkoxycarbonyl may be carried out with a hydride reducing agent, for example, diborane or sodium bis (2-methoxyethoxy) aluminum hydride in an aprotic solvent, for example, tetrahydrofuran. The reduction may be carried out at a temperature of, for example, from 0°-100°C.

g) compounds of formula II in which $R^2$ represents $C_{3-6}$ alkenyl or $C_{3-6}$ alkynyl may be prepared by reacting the corresponding compound in which $R^2$ represents hydrogen with compounds of the formula

$R^7\text{-}CH{=}CH\text{-}CH_2Z$ or $R^7\text{-}C{\equiv}C\text{-}CH_2Z$ in which $R^7$ represents $C_{1-3}$ alkyl and Z represents a halogen;

The reaction may be carried out in the presence of a base, for example, sodium hydride, sodium amide or potassium t-butoxide in a suitable solvent, for example, 1,2-dimethoxyethane, ether, toluene or t-butylalcohol and at a temperature of from -80°-100°C.

h) preparing a compound of the formula II in which $R^2$ represents H or $C_{1-6}$ alkyl, and $R^1$ is a protecting group, by cyclizing a compound of the formula III.

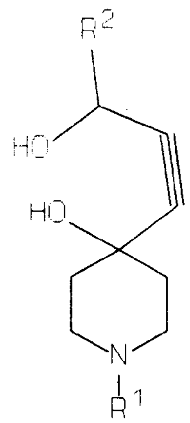

III

This reaction may be carried out by metal catalyzed cylization according to procedures similar to those described by Saimoto, et al [J. Amer. Chem. Soc. 103, 4975-4977 (1981)]. A preferred metal is mercury (II) which may be used in the form of a polymer agent, for example, mercury Nafion-H in aqueous alcoholic solvents, for example, aqueous ethanol, at a temperature of, for example, 0-100°C. Nafion-H is a perfluorinated ion-exchange membrane. Intermediates for the preparation of the ketones may be prepared by a variety of methods, for example

i) preparing a compound of the formula IV,

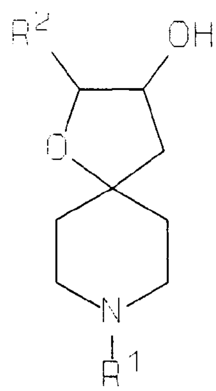

IV

in which

$R^2$ represents H or $C_{1-6}$ alkyl, and $R^1$ represents a protecting group by cyclizing a compound of the formula V.

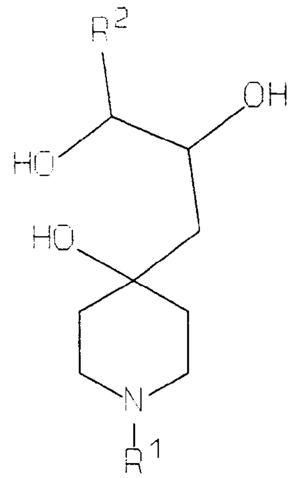

V

Cyclization may be carried out in the presence of a base in a suitable inert solvent or the base may be used in the absence of a solvent. A suitable base may be an organic amine, for example, pyridine.
Cyclization may be facilitated by concomitant derivatization of the alcohol to form a better leaving group, for example, the p-toluene sulfonate ester may be formed by adding p-toluene sulfonyl chloride to the reactants. The reaction may be carried out at a temperature of, for example, 0°-100°C.
The starting materials for the cyclization reactions (h) and (i) can be prepared by a variety of methods which include,
j) preparing a compound of the formula II as defined above by reacting a compound of the formula VI,

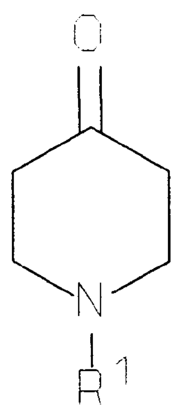

VI

in which $R^1$ is a protecting group with a compound of the formula

$R^8$-CHOH-C≡CH

in which $R^8$ is H or $C_{1-6}$ alkyl.
The reaction may be carried out in the presence of a base, for example, butyl lithium in an inert aprotic solvent, for example, hexane or tetrahydrofuran or mixtures thereof. The reaction may be carried out at a temperature of for example, from -80°C-10°C.
k) preparing a compound of the formula V as defined above by
1) reacting a compound of the formula VI with a compound of the formula $R^9$-CH=CH-$CH_2$MgZ in which $R^9$ represents H or $C_{1-6}$ alkyl and Z represents a halogen, for example, chlorine, bromine or iodine to give a compound of the formula VII,

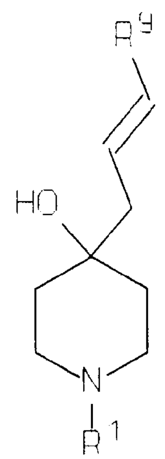

VII

2) epoxidation of the compound of formula VII to the epoxide of formula VIII

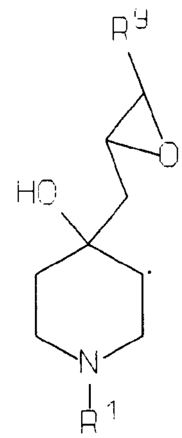

VIII

3) ring-opening of the compound of formula VIII.

The alkylation reaction may be carried out under conventional Grignard reaction conditions, for example, using alkyl magnesium bromide in an inert solvent such as ether or tetrahydrofuran, at a temperature of, for example, from 0°-80°C. The epoxidation reaction may be carried out with an organic peracid or hydrogen peroxide. The organic peracid, for example, m-chloroperbenzoic acid may be used in an inert aprotic solvent, for example, methylene chloride at a temperature of, for example, from 0-50°C. The ring opening of the epoxide may be acid catalyzed, for example, using perchloric acid in a suitable solvent, for example, aqueous tetrahydrofuran, at a temperature of, for example, from 0°-80°C.

1) reduction of a compound of the formula IV in which $R^1$ represents a protecting group, for example ethoxycarbonyl, and $R^2$ is as defined above. Reduction may be carried out with a hydride reducing agent, for example, sodium bis(methoxyethoxy)aluminum hydride in an inert solvent, for example, THF and at a temperature of, for example, from 0°-80°C.

The invention will now be illustrated, but in no way limited, by the following Examples in which all temperatures are in degrees Celsius, THF is tetrahydrofuran, DMSO is dimethyl sulphoxide and ether is diethylether. Solvents which are dried before concentration are dried over magnesium sulfate or sodium sulfate. Stereoisomers are designated by the terms Z and E which are synonomous with the terms cis and trans or syn and anti respectively.

Preparation of Intermediates

Intermediate 1

3-Hydroxy-8-methyl-1-oxa-8-azaspiro[4.5]decane hydrochloride

a) 1-Ethoxycarbonyl-4-hydroxy-4-(2-propenyl)-piperidine

Allyl magnesium bromide was prepared in situ by suspending Mg turnings (21.2g, 0.87mol) in dry ether 700ml) and adding allyl bromide (34.8g, 0.29mol) gradually until the reaction initiated and then at sufficient rate to maintain reflux. The reaction was stirred at room temperature for 1.5 hours.

The reaction was cooled to -15°C with a methanol/ice bath and 1-ethoxycarbonyl-4-piperidinone (25g, 0.146mol) was added in ether (700ml). The reaction was stirred at room temperature for four hours and then left overnight.

The reaction was cooled with an ice bath while quenching with ammonium chloride (360ml of a saturated solution diluted to 1440ml). The reaction was stirred and the phases separated. The aqueous layer was extracted once more with ether and the combined organic layers were washed with brine, dried and stripped. Purification by flash chromatography on silica and elution with $CHCl_3/NH_3$, then $MeOH/CHCl_3/NH_3$ gave the sub-title compound as a yellow oil (17.2g).

b) 1-Ethoxycarbonyl-4-hydroxy-4-(2,3-epoxypropyl)-piperidine

The product of step a) (17.2g, 0.081mol) was dissolved in dry $CH_2Cl_2$ (370ml) under nitrogen. m-Chloroperbenzoic acid (80%, 35g, 0.16mol) was added and the reaction was stirred at room temperature overnight. The white precipitate was removed by suction filtration, and washed with $CH_2Cl_2$. The $CH_2Cl_2$ was washed with 10% sodium sulphite and this was extracted once with $CH_2Cl_2$. The combined organic layers were washed with 10% sodium hydrogen carbonate and this was extracted with $CH_2Cl_2$. The combined organic layers were dried over $Na_2SO_4$ and stripped. The yellow oil obtained was stored under nitrogen in the freezer. Purification by silica flash chromatography, eluting with $MeOH/CHCl_3$ gave the sub-title compound as a yellow oil (11.1g).

c) 1-Ethoxycarbonyl-4-hydroxy-4-(2,3-dihydroxypropyl)piperidine

The partially purified product of step b) (78.9g, 0.34mol) was dissolved in 250ml of THF and 500ml of deionized water. Concentrated $HClO_4$ (50ml) was added and the reaction was stirred overnight. The solution was cooled with an ice bath and neutralized with saturated aqueous $NaHCO_3$. The suspension was then washed with $CH_2Cl_2$, and this was back-extracted with $H_2O$.

The aqueous layers were stripped. The resulting residue was digested with four portions of methanol. These were combined, diluted with $CHCl_3$ and dried over $Na_2SO_4$. The solvents were stripped and the crude was purified by eluting from silica with an ammoniated methanol/$CHCl_3$ gradient. This gave 37.3g of brown oil or 13% for the three steps from the ketone.

d) 8-Ethoxycarbonyl-3-hydroxy-1-oxa-8-azaspiro[4.5] decane

The product of step c) (5.2g, 0.021mol) was dissolved in dry pyridine (60ml), placed under nitrogen and cooled with an ice-bath. Tosyl chloride (4.8g, 0.025mol) was dissolved in pyridine (30ml) and added dropwise. The reaction was heated at 110°C. After 5 hours another 2.2g (0.011mol) tosyl chloride was added in 20ml pyridine at room temperature and the heating was continued overnight.

The pyridine was removed as an azeotrope with three portions of toluene and the residue digested seven times with anhydrous ether. The combined organic extracts were filtered and stripped. Purification on silica, flash column using $MeOH/CHCl_3/NH_3$ gave the sub-title compound as a colourless oil (1.1g). Further ether digestion of the residue, followed by extraction with ether and purification gave a further 1.3g of product.

e) 3-Hydroxy-8-methyl-1-oxa-8-azaspiro[4.5]decane hydrochloride

The product of step d) (1.3g, 5.7mmol) was dissolved in dry THF (60ml), placed under nitrogen and cooled with an ice bath. Vitride (70%, 2.7ml) in THF (30ml) was added dropwise and the reaction stirred at room temperature overnight. Three further portions of Vitride (5ml each) in THF (15ml each) were added dropwise to the cooled solution and after each portion the solution was stirred for several hours. The reaction was cooled

again and treated with 5% NaOH until evolution of hydrogen ceased.

The addition of NaOH was continued at room temperature until a sticky white paste had precipitated. The THF was decanted, suction filtered, and the paste washed once with THF. The solvent was then dried and stripped, and the paste washed once with THF. Purification by silica flash column using $MeOH/CHCl_3$ gave a viscous yellow oil (1.1g) which partially solidified on standing to white needles. The solid was taken up in isopropyl alcohol and the solution cooled and acidified with HCl/ethanol. This gave a white precipitate which was collected and washed with cold ether to yield the title compound as the hydrochloride salt (0.47g), mp 228-229°C.

Intermediate 2

8-Methyl-1-oxa-8-azaspiro[4.5]decan-3-one

Oxalyl chloride (1.9g, 14.3mmol) was dissolved in dry $CH_2Cl_2$ (150ml), placed under $N_2$ and cooled to -60°C with a dry ice/acetone bath. Dry DMSO (2.2g, 29mmol) in dry $CH_2Cl_2$ (30ml) was added in slow drops. The reaction was stirred for 10 minutes. The free base of Example 1 (1.5g, 8.8mmol) in $CH_2Cl_2$ (100ml) was added in slow drops, the temperature being maintained below -60°. The reaction was stirred for 20 minutes and then was treated dropwise with diisopropylethylamine (9.0g, 67.5mmol).

The bath was removed and the reaction allowed to warm somewhat. The solution was treated with distilled water (150ml) in rapid drops. The layers were separated and the aqueous layer was extracted three times with $CH_2Cl_2$. A little saturated $Na_2CO_3$ was added and two more $CH_2Cl_2$ extractions were done.

The organic layers were dried with $Na_2SO_4$ and stripped. Purification by silica flash chromatography, using $MeOH/CHCl_3/NH_3$ gave the title product as a yellow oil (1.5g).

Intermediate 2A

8-Methyl-1-oxa-8-azaspiro[4.5]decan-3-one maleate

The yellow oil obtained in Intermediate 2 was taken up in ethyl acetate, the solution cooled in an ice bath and maleic acid/ethyl acetate was added. The title compound was obtained as an off-white powder, mp 127-128.5°.

| | C | H | N |
|---|---|---|---|
| Theory: | 54.73 | 6.71 | 4.91 |
| Found: | 54.33 | 6.55 | 4.77 |

NMR (DMSO): a 6.1(2H), 4.0(2H), 3.3(4H,m), 2.8(3H), 2.0(4H,m)
IR (KBr): 2675, 1760, 1580, 1360, 1380, 930 $cm^{-1}$ $(M+1)^+ = 170$

Intermediate 3

8-Methyl-1-oxa-8-azaspiro[4.5]decan-3-one oxime

The compound of Intermediate 2 (0.6g, 3.5mmol) was dissolved in methanol (50ml) and placed under nitrogen. Hydroxylamine hydrochloride was added and the reaction was stirred at room temperature overnight.

The solvent was evaporated, and the residue taken up in $CHCl_3$ and treated with saturated $Na_2CO_3$. The layers were separated and the basic layer extracted with three more portions of $CHCl_3$. The combined organic extracts were dried with $Na_2SO_4$ and stripped. Purification by silica flash chromatography using $MeOH/CHCl_3/NH_3$ gave the title compound as a pale yellow solid.

Intermediate 4

3-Hydroxy-2,8-dimethyl-1-oxa-8-azaspiro[4.5]decane

a) 4-(3-Hydroxy-1-butynyl)-1-ethoxycarbonyl-4-piperidinol

A solution of 3-butyn-2-ol (42g, 0.6mol) in dry THF (800ml) under nitrogen was cooled with a dry-ice/acetone bath. n-Butyl lithium (390ml of 2.5M and 230ml of 1.5M) in hexane was added rapidly, dropwise. The suspension became gelatinous and another 800mls of dry THF was added. The reaction was stirred at approximately -78°C for 1 hour and then at 0°C for 20 minutes. The reaction was cooled to -78°C and 1-ethoxycarbonyl-4-piperidinone (104g, 0.607mol) in 300ml of dry THF was added rapidly dropwise. The reaction mixture was allowed to warm to room temperature and then stirred overnight. The reaction mixture was cooled in an ice-bath, diluted with THF (1.51), decomposed with saturated $NH_4Cl$, and the THF layer was washed three times with 200ml of saturated ammonium chloride. The aqueous layers were backwashed with THF (200ml x 3). The organic layers were combined and dried over $MgSO_4$ and evaporated to give an oil. A one-fifth portion of the oil was purified by flash chromatography on silica gel and elution with 30% ethyl acetate/ether afforded the desired product diol (19.7g).

b) 8-Ethoxycarbonyl-2-methyl-1-oxa-8-azaspirodecan-3-one

The diol of step a) (19.7g, 0.082mol) was dissolved in ethanol (82ml) and water (7.4g, 0.41mol). Mercury/Nafion NR50 (41g) prepared according to Y Saimoto et al (Bull Chem Soc Japan, 56, 3078 (1983)) was added and the slurry was stirred in a closed flask for three days. The resin was filtered off and washed with methylene chloride. The combined filtrates were evaporated. The residue was dissolved as far as possible in ether and filtered through a bed of silica gel to clarify the solution. The filtrate was evaporated to give the ketone as a yellow oil (13.3g).

Intermediate 5

2,8-Dimethyl-1-oxa-8-azaspiro[4.5]decan-3-one

a) 8-Ethoxycarbonyl-2-methyl-1-oxa-8-azaspiro[4.5]-decan-3-one ethylene ketal

8-Ethoxycarbonyl-2-methyl-1-oxa-8-azaspiro[4.5]-decan-3-one (23g, .0963 mol), ethylene ketal (50ml) and p-toluenesulfonic acid (1g) were suspended in toluene (500ml) and refluxed for 3 hours while water and ethylene glycol were collected in a Dean and Stark trap. The solution was cooled, washed with water and the aqueous layer was backwashed with chloroform. The combined solvent layers were dried and concentrated to give the title ketal as a thick oil (25.8g).

b) 2,8-Dimethyl-1-oxa-8-azaspiro[4.5]decan-3-one ethylene ketal

The carbamate-ketal from step (a) (10g, .035mol) in THF (50ml) was added at room temperature during 30 mins. to a solution of Vitride (0.105mol) in THF (150ml). The reaction was stirred for 1 hour then decomposed by the addition of 20ml of 20% aqueous THF then additional water until a clear supernatant layer formed. The solvent layer was separated and evaporated in vacuo. The residue was chromatographed on silica gel and eluted with 20% $MeOH/CH_2CL_2$ to give the dimethyl-ethylene ketal as a thick oil (1414g). A sample of the oil (0.5g) was converted to the maleate salt in ether and crystallized from methylene chloride/ether to give the salt (0.24g), mp 105-108°C.

| | C | H | N |
|---|---|---|---|
| Theory: | 55.96 | 7.34 | 4.08 |
| Found: | 56.10 | 7.48 | 4.10 |

c) 2,8-Dimethyl-1-oxa-8-azaspiro[4.5]decan-3-one maleate

The ketal base from step (c) (14.4g, 0.0634mol) dissolved in 1.25N.HCL (100ml) was heated at 50°C for 4 hours. The reaction was cooled, basified with saturated aqueous $Na_2CO_3$ solution and the precipitated base was extracted into chloroform. The chloroform solution was dried and the solvent evaporated in vacuo to give the ketone as an oil (13.35g).

A sample of the oil (4.46g) was converted to the maleate salt in ether. The precipitated salt was recrystallized twice from ethyl acetate to give the title product (3.11g), mp 137.5-139°C.

| | C | H | N |
|---|---|---|---|
| Theory: | 56.18 | 7.07 | 4.68 |
| Found: | 56.23 | 7.14 | 4.70 |

Intermediate 6

2,8-Dimethyl-1-oxa-8-azaspiro[4.5]decan-3-one oxime fumarate

Following essentially the same procedure as described for Intermediate 3 and substituting 2,8-dimethyl-1-oxa-8-azaspiro[4.5[decan-3-one (1.4g, 7.7mmol) for 8-methyl-1-oxa-8-azaspiro[4.5]decan-3-one afforded the free base as an oil (2.1g). The oil was dissolved in ethyl acetate and diluted with a saturated solution of fumaric acid in ether. The precipitated solid was filtered and dried to give the title product (1.5g).

| | C | H | N |
|---|---|---|---|
| Theory: | 53.49 | 7.05 | 8.91 |
| Found: | 53.48 | 7.24 | 9.55 |

Preparation of Examples

Example 1

Preparation of (E)-and(Z)-2,8-Dimethyl-1-oxa-8-azaspiro[4.5]decan-3-one oxime methyl ether maleates

2,8-Dimethyl-1-oxa-8-azaspiro[4.5]decan-3-one (5g, 0.027 mol) was dissolved in absolute ethanol (200mL) and pyridine (0.44mL) was added. Methoxyamine hydrochloride (2.5g, 0.03 mol) was added and the reaction mixture was stirred overnight. The ethanol was evaporated and the residue was dissolved in $CHCl_3$ and partitioned with aqueous sodium carbonate (saturated). The chloroform layer was dried ($MgSO_4$) and evaporated. The residual mixture of isomers was purified by gradient chromatography on silica gel and eluting with $CHCl_3$/EtOAc/MeOH(49/49/2--43/43/14) to give essentially pure fractions of high $R_F$ and low $R_F$ isomers.

The higher $R_F$ isomer (1.8g) was dissolved in ethyl acetate and treated with maleic acid (1.1 eq.). Diethyl ether was added to precipitate a white solid which was dried at 75°C in vacuo to give (E)-2,8-Dimethyl-1-oxa-8-azaspiro[4.5]decan-3-one oxime methyl ether maleate (1.8g), mp 146-147°C.

| | C | H | N |
|---|---|---|---|
| Theory: | 54.87 | 7.37 | 8.53 |
| Found: | 54.92 | 7.36 | 8.58 |

The lower $R_F$ isomer (0.51g) was dissolved in ethyl acetate and treated with maleic acid (1.1 eq). The precipitated solid (0.45g) was collected and combined with a similarly prepared material to give a total of 0.6g which was dried at 75°C in vacuo to give (Z)-2,8-Dimethyl-1-oxa-8-azaspiro[4.5]decan-3-one oxime methyl ether

maleate, mp 146.5-149.5°C.

| | C | H | N |
|---|---|---|---|
| Theory: | 54.87 | 7.37 | 8.53 |
| Found: | 54.24 | 7.26 | 8.46 |

Example 2

N-Ethoxycarbonyl-2,8-dimethyl 1-oxa-8-azaspiro[4.5]decan-3-one hydrazone fumarate

2,8-Dimethyl-1-oxa-8-azaspiro[4.5]decan-3-one (3g, 0.017 mol) and ethyl carbazate (1.56g) were dissolved in absolute EtOH (175mL) and acidified with HCl/EtOH to pH4. The reaction was stirred overnight at room temperature and then the ethanol was evaporated. The residue was dissolved in $CHCl_3$ and partitioned with aqueous $Na_2CO_3$. The chloroform layer was dried ($Na_2SO_4$) and concentrated. The residue was purified by chromatography on silica gel and eluting with ammoniated 2-10% MeOH/$CHCl_3$ to give 1.4g of an oil. The oil was dissolved in diethyl ether and acidified with a solution of fumaric acid in diethyl ether. The precipitated solid was recrystallized from ethanol/diethyl ether to give a hygroscopic solid which was dried under high vacuum at 50°C to give the title compound as a solid mp 103.5 - 104.5°C.

| | C | H | N |
|---|---|---|---|
| Theory: | 52.98 | 7.06 | 10.90 |
| Found: | 52.34 | 7.32 | 11.29 |

Example 3

2,8-Dimethyl-1-oxa-8-azaspiro[4.5]decan-3-one 0-[(dimethylamino)carbonyl]oxime fumarate

A solution of 2,8-Dimethyl-1-oxa-8-azaspiro [4.5]decan-3-one oxime (1g, 5 mmol) dissolved in dry THF containing sodium hydride (0.24g of a 60% oil suspension) was stirred until hydrogen evolution had ceased. (Dimethylamino)carbonyl chloride (0.66mL) was added and the reaction was stirred at room temperature overnight, then it was heated at reflux for 5 hours. The reaction was treated with aliquots of (dimethylamino)carbonyl chloride and further heating until the reaction was complete. The solvent was evaporated and the residue was dissolved in chloroform, partitioned with aqueous sodium carbonate and the chloroform layer was concentrated to dryness. The residue was chromatographed on silica gel and eluted with ammoniated 2-10% MeOH/$CHCl_3$ to give an oil (1g). The oil was treated with maleic acid (0.43g) in ethyl acetate and concentrated to dryness. The residue was dissolved in isopropanol/ethylacetate and concentrated under vacuum until turbidity occurred. A small quantity of oil separated and the supernatant solution was decanted and concentrated. The free base was recovered from the maleate salt by treatment with aqueous sodium carbonate and further purified by chromatography. The recovered base (0.3g) was then treated with fumaric acid in ethylacetate/diethyl ether to give the fumarate salt of the title compound, 0.24g, mp. 165-169°C.

| | C | H | N |
|---|---|---|---|
| Theory: | 52.98 | 7.06 | 10.90 |
| Found: | 52.89 | 7.01 | 10.50 |

solid was filtered and dried to give the title product (1.5g).

| | C | H | N |
|---|---|---|---|
| Theory: | 53.49 | 7.05 | 8.91 |
| Found: | 53.48 | 7.24 | 9.55 |

## Claims

1. A compound of the formula I,

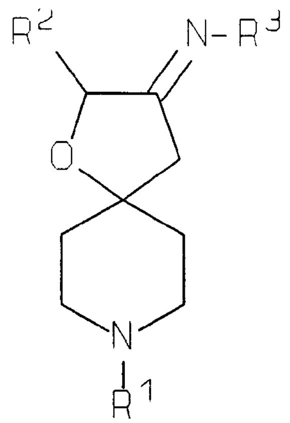

I

wherein
$R^1$ represents hydrogen or alkyl $C_{1-3}$,
$R^2$ represents hydrogen or alkyl $C_{1-6}$, alkenyl $C_{3-6}$ or alkynyl $C_{3-6}$,
$R^3$ represents $OCH_3$, $OCONR^4R^5$ or $NHCOOR^6$,
$R^4$, $R^5$, and $R^6$ represent hydrogen or $C_{1-6}$ alkyl, provided that when $R^3$ represents $OCH_3$, then $R^1$ and $R^2$ both represent methyl,
and salts and solvates thereof.

2. A compound according to claim 1 in which $R^1$ represents hydrogen or methyl.

3. A compound according to claim 1 or claim 2 in which $R^2$ represents hydrogen or methyl.

4. A compound according to any one of the preceding claims in which $R^3$ represents $OCH_3$.

5. A compound according to any one of the preceding claims in which the carbon-nitrogen double bond has the syn configuration.

6. A compound according to claim 1 which is
(E)-2,8-Dimethyl-1-oxa-8-azaspiro[4.5]decan-3-one oxime methyl ether,
(Z)-2,8-Dimethyl-1-oxa-8-azaspiro[4.5]decan-3-one oxime methyl ether,
N-Ethoxycarbonyl-2,8-dimethyl 1-oxa-8-azaspiro[4.5]decan-3-one hydrazone,
2,8-Dimethyl-1-oxa-8-azaspiro[4.5]decan-3-one 0-[(dimethylamino)carbonyl]oxime,
or a pharmaceutically acceptable salt thereof.

7. The use of a compound of formula I, as defined in claim 1, or a pharmaceutically acceptable salt thereof, as a pharmaceutical.

8. A pharmaceutical composition comprising a compound of formula I, as defined in Claim 1, or a pharmaceutically acceptable salt thereof, in admixture with a pharmaceutically acceptable adjuvant, diluent or

carrier.

**9.** The use of a compound of formula I, as defined in claim 1, or a pharmaceutically acceptable salt thereof, in the manufacture of a medicament for use in the treatment of neurological disorders.

**10.** A process for the preparation of compounds of formula I, as defined in claim 1, or pharmaceutically acceptable salts thereof, which comprises

a) preparing a compound of formula I in which $R^3$ represents $OCONR^4R^5$ and $R^4$ and $R^5$ are as defined above,

by reacting the corresponding compound of formula I in which $R^3$ is OH with the corresponding aminocarbonyl halide, or

b) preparing a compound of formula I in which $R^3$ represents $OCH_3$ or $NHCOOR^6$ and $R^6$ is as defined above,

by reacting the corresponding compound of formula II

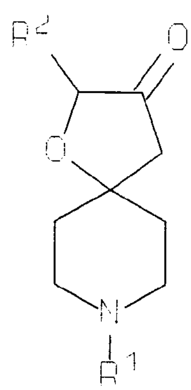

II

in which $R^1$ and $R^2$ are as defined above with the corresponding nitrogen nucleophile of the formula $H_2N\text{-}OCH_3$ or $H_2NNHCOOR^6$, or

c) removing a protecting group from a compound of formula I in which the amino group is protected, or

d) preparing a pharmaceutically acceptable salt of a compound of formula I by treating a compound of formula I with an appropriate acid or preparing a free base of a compound of formula I by treating the acid addition salt with a stronger base.

European Patent Office

# EUROPEAN SEARCH REPORT

Application Number

EP 91 31 1743

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | EP-A-0 311 313 (YAMANOUCHI PHARMACEUTICAL CO.)<br>* page 12, line 9 - page 12, line 35; claims 1,9 *<br>--- | 1,8,9 | C07D491/10<br>A61K31/445<br>//(C07D491/10, 307:00,221:00) |
| P,X | CHEMICAL ABSTRACTS, vol. 115, no. 19, 11 November 1991, Columbus, Ohio, US; abstract no. 207873A, S. TSUKAMOTO ET AL.: 'Preparation of heterocyclic spiro derivatives as muscarinic acetylcholine receptor M1 agonists.' page 1010 ;<br>& JP-A-3153690 (YAMANOUCHI PHARMACEUTICAL CO. )<br>* abstract *<br>--- | 1,8,9 | |
| D,P, X | WO-A-9 015 804 (FISONS CORPORATION)<br>* page 3, line 17 - page 3, line 34; claims 1,9 *<br>----- | 1,8,9 | |
| | | | TECHNICAL FIELDS SEARCHED (Int. Cl.5 ) |
| | | | C07D<br>A61K |

The present search report has been drawn up for all claims

| Place of search | Date of completion of the search | Examiner |
|---|---|---|
| THE HAGUE | 25 MARCH 1992 | VOYIAZOGLOU D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)